# FASCICULE DE BREVET EUROPEEN

(11) **EP 0 574 277 B1**
(45) Date de publication et mention de la délivrance du brevet: **22.10.1997**
(21) Numéro de dépôt: 93401128.9
(22) Date de dépôt: 30.04.1993
(51) Int. Cl.: C11D 3/32, C11D 1/52

(54) **Utilisation d'un amide comme agent épaississant et composition le contenant**
Verwendung eines Amids als Verdickungsmittel und dieses enthaltende Zusammensetzung
Use of an amide as thickening agent and composition containing the same

(30) Priorité: 22.05.1992 FR 9206284
(43) Date de publication de la demande: 15.12.1993
(73) Titulaire: WITCO, F-75008 Paris (FR)
(72) Inventeur: Lefebvre, Ginette, F-27670 Bosc Roger en Roumois (FR); Fiquet, Line, F-76100 Rouen (FR)
(74) Mandataire: Peuscet, Jacques

(56) Documents cités:
- EP-A- 0 285 768
- GB-A- 921 036
- US-A- 3 856 711
- DATABASE WPI Week 7901, Derwent Publications Ltd., London, GB; AN 79-00918B

## Description

La présente invention concerne, d'une part, un agent épaississant constitué par un amide et utilisable dans des compositions contenant au moins un agent tensioactif, d'autre part, des compositions contenant ledit amide comme agent épaississant.

Les compositions contenant au moins un agent tensioactif, dont on souhaite augmenter la viscosité, peuvent être des produits cosmétiques, notamment des shampooings, des gels de douche ou des crèmes mais aussi des détergents liquides à usage domestique, notamment des liquides pour vaisselle, des gels nettoyants pour cuvette de WC, des nettoyants pour carrelage, ou des lessives liquides.

Il est bien connu que la présence de tensioactifs dans une composition diluée conduit à une formulation de faible viscosité. Or, lorsque la composition a une faible viscosité, le contact avec la surface sur laquelle elle est utilisée est souvent mauvais ; de plus, le produit s'écoulant trop facilement, il n'a pas le temps d'agir et l'utilisateur a tendance à en utiliser une quantité trop élevée, ce qui augmente la consommation.

Pour remédier à ce problème, on a proposé l'addition aux compositions, de nombreux épaississants. Il est connu d'utiliser comme épaississant des amides d'acides gras, par exemple par US-A-3 856 711, EP-A-0 285 768 et JP-A-53133107. Parmi ces épaississants, on a proposé d'utiliser des diéthanolamides qui, étant préparés à partir d'amines secondaires, ont l'inconvénient de présenter une forte teneur en nitrosamines. Or, il est connu que les nitrosamines sont à éviter, compte-tenu de leur potentiel cancérogène. On a cependant largement utilisé le diéthanolamide de coprah car il a l'avantage d'être sous forme d'un liquide manipulable à température ambiante. Mais ce composé, outre sa teneur en nitrosamines, présente l'inconvénient de ne permettre de préparer des compositions de viscosité élevée qu'avec des concentrations élevées en amide. On a également proposé l'utilisation de monoisopropanolamides de palme, de coprah, de stéarine et d'oléine qui ont l'avantage de ne pratiquement pas contenir de nitrosamines puisqu'ils sont préparés à partir d'amines primaires, mais ces amides sont des solides à température ambiante et sont donc difficilement manipulables.

De plus, avec les amides utilisés jusqu'à présent, il était nécessaire, pour obtenir une composition ayant une viscosité élevée, d'ajouter une quantité importante d'un sel minéral, en particulier de chlorure de sodium ou de sulfate de magnésium. En effet, il est connu que, pour une quantité déterminée d'amide épaississant, la viscosité croît avec la teneur en sel minéral jusqu'à un maximum, pour décroître ensuite. Avec les amides utilisés jusqu'à présent, la viscosité optimale n'est généralement atteinte qu'en présence de quantités assez élevées de sel minéral (NaCl, Mg SO₄). Or la présence d'un sel minéral présente l'inconvénient de donner un produit moins stable à basse température et moins doux à l'utilisation, ces inconvénients étant d'autant plus sensibles que la teneur en sel minéral est plus élevée.

Selon la présente invention, on a trouvé que l'utilisation d'un amide déterminé, le monoisopropanolamide d'acide isostéarique, liquide à température ambiante, permet de préparer facilement des compositions contenant au moins un tensioactif et ayant une viscosité élevée en présence d'une faible quantité de sel minéral, en particulier de chlorure de sodium ou de sulfate de magnésium. On peut ainsi préparer des compositions mieux adaptées à l'utilisation.

La présente invention a donc pour premier objet l'utilisation comme agent épaississant, dans une composition contenant au moins un agent tensioactif, d'une quantité efficace de l'amide de formule : 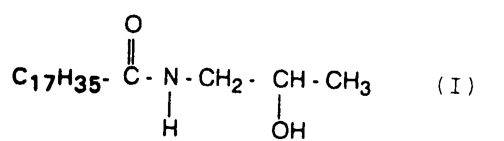 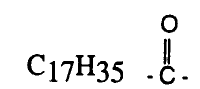 étant un radical isostéaroyle.

On a constaté que le monoisopropanolamide d'acide isostéarique a, en plus de son excellent pouvoir viscosifiant, des propriétés adoucissantes, lubrifiantes et émulsionnantes et un effet renforçateur de mousse. Par ailleurs, le monoisopropanolamide d'acide isostéarique a l'avantage de permettre de formuler des compositions contenant des tensioactifs à température ambiante car il est liquide et manipulable à cette température, donc facile à introduire avec des tensioactifs.

On obtient l'amide de formule (I) comme produit de condensation de l'acide isostéarique et de l'amino-1 propanol-2 (également appelé 2-hydroxypropylamine, monoisopropanolamine ou MIPA) ; il a, à 20° C, l'aspect d'un liquide limpide à légèrement trouble se clarifiant entre 30 et 40° C : il est, de ce fait, facile à introduire en formulation avec des tensioactifs. La densité de cet amide, mesurée à 40° C, est de 0,904 ; sa viscosité, à 40° C, est de 320 mPa.s

L'invention a, en outre, pour objet un agent épaississant utilisable dans des compositions contenant au moins un agent tensioactif, ledit agent renfermant au moins un amide d'acide gras, caractérisé par le fait qu'il contient l'amide de formule (I). Cet agent épaississant contient de préférence au moins 90 % en poids de l'amide de formule (I).

L'agent épaississant selon l'invention répond avantageusement aux spécifications suivantes :
- Aspect à 25° C liquide limpide à légèrement trouble
- Densité à 40° C 0,900 - 0,908
- Viscosité à 40° C 310 à 330 mPa.s
- Couleur à 40° C (mesurée en unités Gardner) ≤ 5
- Indice d'acide (en mg KOH/g) ≤ 5
- Teneur en amine libre (en % en poids) ≤ 1
- Teneur en estéramide (en % en poids) ≤ 5
- pH mesuré en solution à 1 % en poids dans un mélange eau/isopropanol (50/50 en volume) 8 à 9
- Teneur en eau ≤ 0,5

La présente invention a également pour objet une composition liquide ou pâteuse contenant au moins un tensioactif et au moins une quantité efficace de l'agent épaississant selon l'invention, tel que ci-dessus défini.

L'agent épaississant selon l'invention est compatible avec la plupart des tensioactifs et des savons. Dans la composition selon l'invention, le(s) tensioactif(s) peut (peuvent) avantageusement être choisi(s) dans le groupe formé par :
- les alkylsulfates alcalins (en particulier de sodium), d'ammonium, de magnésium, ou d'alcanolamines, en particulier de monoéthanolamine ou de triéthanolamine ;
- les alkyléthersulfates alcalins (en particulier de sodium), d'ammonium, de magnésium ou d'alcanolamines, en particulier de monoéthanolamine ou de diéthanolamine ;
- les alkylbenzène sulfonates alcalins ou d'alcanolamines ;
- les alphaoléfine sulfonates alcalins ;
- les alkyl amidosulfosuccinates alcalins ;
- les alkyl sulfosuccinates de sodium;
- les savons de sodium, potassium ou triéthanolamine ;
- les bétaïnes et sulfobétaïnes ;
- les tensioactifs amphotères dérivés d'imidazoline ;
- les alkylpolyglycoléthers ;
- les alkylarylpolyglycoléthers ;
- les esters gras de polyols, en particulier de polyéthylèneglycol, de sorbitol ou de sorbitol éthoxylés.

Les compositions selon l'invention contiennent des quantités d'agent épaississant selon l'invention, qui varient selon la nature et la quantité d'agent(s) tensioactif(s) utilisé(s) et selon l'usage auquel est destinée la composition. Ces quantités sont généralement comprises entre 0,1 et 2,5 % en poids par rapport au poids total de la composition, de préférence entre 0,5 et 1,5 %.

Les compositions épaissies selon l'invention contenant des amides contiennent également le plus souvent un sel minéral, en particulier un chlorure ou un sulfate alcalin ou alcalino-terreux, plus particulièrement le chlorure de sodium ou le sulfate de magnésium ; l'utilisation de l'amide de formule (I) comme constituant essentiel de l'agent épaississant selon l'invention permet de diminuer la quantité de chlorure de sodium ou de sulfate de magnésium nécessaire pour obtenir une viscosité donnée avec une quantité donnée d'amide ; dans la pratique la quantité de chlorure de sodium ou sulfate de magnésium est donc choisie pour optimiser la viscosité selon la quantité d'amide de formule (I) présente dans la composition. Selon l'invention, la composition contient par exemple entre 0,25 et 10 % en poids de chlorure de sodium pour une quantité de monoisopropanolamide d'acide isostéarique comprise entre 0,1 et 2,5 % ; de préférence, elle contient de 0,75 à 1,75 % en poids de NaCl pour 0,5 à 1 % d'amide de formule (I).

La composition selon l'invention peut constituer un shampooing ou une crème, un gel de douche ou un détergent liquide pour le nettoyage de la vaisselle, pour le nettoyage des WC, ou pour le nettoyage des carrelages, ou une lessive liquide.

Le monoisopropanolamide d'acide isostéarique peut être préparé par réaction de l'acide isostéarique et de la monoisopropanolamide par tout procédé connu de préparation d'un amide. On utilise de préférence un procédé dans lequel on fait réagir une quantité stoechiométrique ou un léger excès d'isopropanolamine sur l'acide isostéarique en présence d'acide phosphorique comme catalyseur. Cependant, selon le procédé utilisé, on peut obtenir un produit trop coloré, c'est-à-dire ayant une coloration en unités Gardner supérieure à 5, une teneur en esteramide, obtenu comme produit secondaire, trop élevée ainsi qu'une teneur en amine libre trop élevée, pour que l'amide de formule (I) puisse, sans inconvénient, être utilisé pour constituer un agent épaississant convenable en vue de son utilisation dans des compositions selon l'invention.

Le procédé de préparation de l'agent épaississant selon l'invention, décrit ci-après permet d'obtenir un produit ayant une coloration en unités Gardner de 5 au maximum, une teneur en esteramide de 5 % en poids au maximum et une teneur en amine libre de 1 % en poids au maximum.

Selon ce procédé :
a) on introduit de l'acide isostéarique dans un réacteur traversé par un courant de gaz inerte et on le chauffe à une température comprise entre 40 et 70° C;
b) on ajoute ensuite, dans ledit réacteur, un antioxydant, et on maintient le mélange sous agitation pendant 0,5 à 2 heures;
c) on ajoute progressivement, dans le réacteur, de la monoisopropanolamine, on laisse la température s'élever pour arriver dans la plage comprise entre 90 et 110° C, et on maintient cette température en agissant sur le débit d'introduction de la monoisopropanolamine jusqu'à ce que l'on ait introduit entre 1,00 et 1,10 fois le nombre de moles d'acide placé dans le réacteur à l'étape a)
d) on maintient la température, on introduit lentement dans le réacteur de l'acide phosphorique en une quantité comprise entre 2 et 5 parties en poids pour 10 000 parties en poids d'acide placé dans le réacteur à l'étape a) et on porte la température à une valeur comprise entre 145 et 170° C ;
e) on maintient la température jusqu'à ce que l'indice d'acide soit inférieur à 5 (mg KOH/g) ;
f) lorsque l'indice d'acide est inférieur à 5, on ajoute une seconde quantité d'antioxydant, on établit une pression réduite de 4,0 à 1,9 x 10³ Pa en maintenant l'atmosphère de gaz inerte, pour éliminer l'excès d'amine ;
g) puis on abaisse la température au dessous de 75° C en maintenant l'atmosphère de gaz inerte et on laisse revenir à température ambiante.

Les exemples donnés ci-après, à titre illustratif et nullement limitatif, permettront de mieux comprendre l'invention.

### Exemple 1 - Préparation d'un agent épaississant à base de monoisopropanolamide d'acide isostéarique

On met en oeuvre les produits suivants (les quantités sont données en g) :
- Acide isostéarique 668
- Monoisopropanolamine 186
- Acide phosphorique à 85 % 0,26
- 2,6-di-tert-butyl-p-crésol (antioxydant) 1,92

On purge un réacteur de 1 litre à l'azote et on y introduit tout l'acide isostéarique. On chauffe l'acide à une température de 60-70°C sous un courant d'azote ayant un débit de 0,4 l/h. On ajoute ensuite la moitié de l'antioxydant et on maintient sous agitation pendant 1 heure.

On ajoute la monoisopropanolamine (MIPA) en filet. La réaction est exothermique et la température du mélange réactionnel s'élève. On la laisse s'élever jusqu'à 100° C pour fluidifier le mélange réactionnel et on la maintient à 100° C en agissant sur le débit d'introduction de la MIPA.

Après introduction de la MIPA, on introduit lentement à 100° C l'acide phosphorique. On double le débit d'azote puis on chauffe à 155-160° C.

On maintient cette température pendant toute la réaction jusqu'à ce que l'indice d'acide (en mg de KOH/g) soit inférieur à 5. On contrôle l'évolution de la réaction en prélevant toutes les heures et demie, des échantillons sur lesquels on détermine à la fois l'indice d'acide et l'indice d'alcalinité. L'indice d'alcalinité exprimé en mg de KOH/g doit être en permanence supérieur de 10 points à l'indice d'acide. Si ce n'est pas le cas, on ajuste l'indice d'alcalinité par addition de MIPA.

Pendant la réaction, on augmente progressivement le débit d'azote de façon qu'il atteigne, en fin de réaction, dix fois le débit initial utilisé au moment du chauffage de l'acide isostéarique. On favorise ainsi l'entraînement de l'eau formée.

Lorsque l'indice d'acide a atteint une valeur inférieure à 5 (en mg de KOH/g), on ajoute la seconde moitié de l'antioxydant.

On établit une pression de 2,66 x 10³ Pa dans le réacteur sous un balayage d'azote de 4,3 l/h pour éliminer par distillation l'excès de MIPA. La température de distillation de l'amine est très importante car, pour des températures supérieures, on obtiendrait un produit ayant une teneur trop élevée en estéramide. On maintient la pression réduite jusqu'à ce que l'indice d'alcalinité soit inférieur à 0,1 meq/g.

Pour finir, on refroidit le mélange réactionnel à 60° C, sous un courant d'azote de 0,9 l/h et on laisse ensuite revenir à température ambiante.

On obtient ainsi, avec un rendement de 93 % par rapport aux matières premières mises en oeuvre, un agent épaississant répondant aux spécifications suivantes :
- Aspect à 25°C liquide limpide à légèrement trouble
- Couleur en unités Gardner (1) 4,5
- Indice d'acide (mg KOH/g) 4,2
- Teneur en amine libre (% en poids) 0,8
- Teneur en estéramide (% en poids) 4,1
- Teneur en eau (% en poids) 0,05
- Point de cristallisation (° C) 10
- pH en solution à 1 % dans un mélange eau/isopropanol 50/50 en volume 8,8
- Viscosité à 40° C (mPa.s) 320
- Densité à 40° C 0,904
(1) La mesure se fait par comparaison avec des disques de coloration en unités normalisées Gardner de 1 à 18.

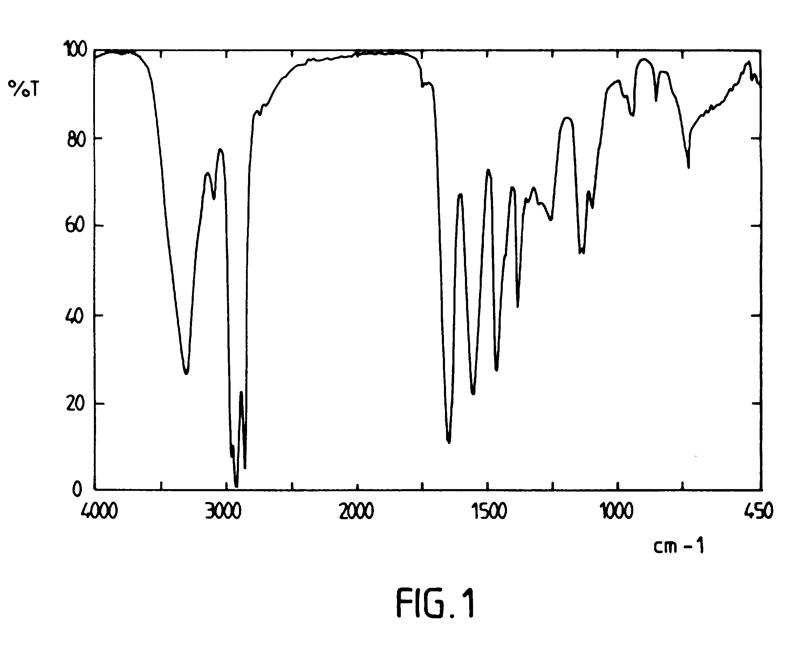

Le spectre infra-rouge du composé obtenu est illustré sur la figure 1 annexée, sur laquelle les longueurs d'onde en cm⁻¹ sont données en abscisse et le pourcentage de transmission en ordonnée. Ce spectre confirme que le produit obtenu est bien essentiellement constitué par le monoisopropanolamide d'acide isostéarique.

### Exemple 2 - Préparation d'un shampooing

On a préparé un shampooing contenant des quantités variables de chlorure de sodium ayant la formulation suivante (en % en poids) :
- Tensioactif à base de sulfosuccinate vendu sous la dénomination commerciale "EMCOL 1484" par la société "WITCO" 15
- Tensioactif à base d'alkyléthersulfate de sodium vendu sous la dénomination commerciale "NEOPON LOS/NF" par la société "WITCO" 20
- Agent épaississant 1
- Chlorure de sodium 1 à 10
- Eau qsp 100

L'agent épaississant utilisé est soit l'agent épaississant selon l'invention obtenu selon l'exemple 1, soit, à titre comparatif, le monoisopropanolamide de palme vendu sous la marque "WITCAMIDE PPA" par la Société "WITCO" et le monoisopropanolamide de coprah vendu sous la marque "WITCAMIDE CPA" par la société "WITCO".

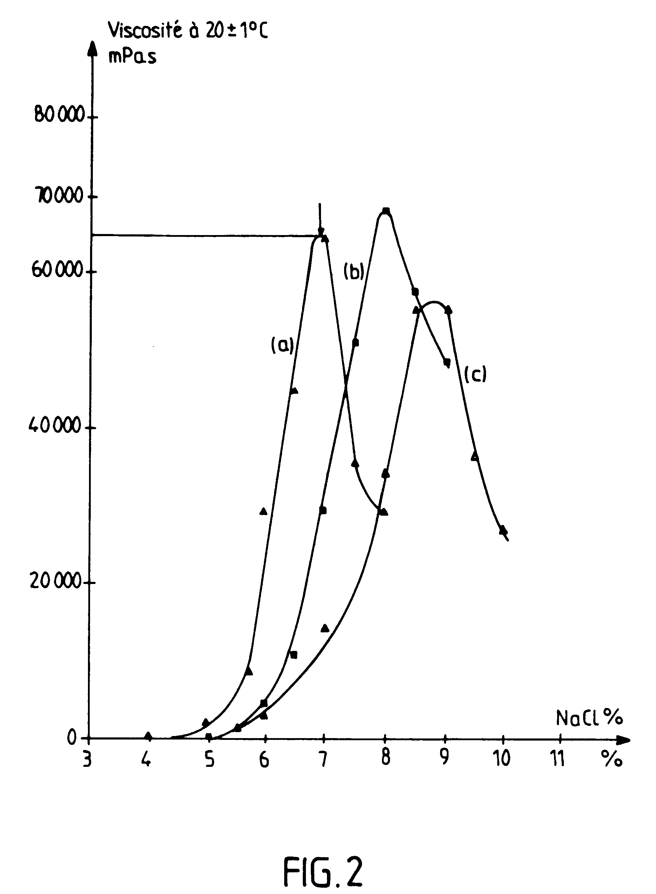

On a mesuré à 20° C la viscosité avec un viscosimètre "Brookfield RVT" des différentes formulations obtenues. Les résultats de ces mesures sont indiqués sur la figure 2 annexée, dans laquelle le pourcentage de chlorure de sodium est porté en abscisse et la viscosité mesurée à 20° C en mPa.s, en ordonnée. La courbe (a) correspond à l'agent épaississant selon l'invention, la courbe (b) est celle obtenue avec la "WITCAMIDE PPA" et la courbe (c) est celle obtenue avec la "WITCAMIDE CPA".

Sur cette figure 2, on voit que la viscosité maximale que l'on peut obtenir avec l'agent épaississant selon l'invention est voisine de celle obtenue avec les "WITCAMIDE CPA et PPA" et que, si on compare les parties ascendantes des courbes a, b et c on peut toujours, avec l'agent épaississant selon l'invention, obtenir une viscosité donnée avec une quantité plus faible de NaCl.

On a également mesuré le point de trouble des compositions ci-dessus contenant la "WITCAMIDE CPA", la "WITCAMIDE PPA" et l'agent épaississant selon l'invention pour des teneur en NaCl de 7% et 8%. Les résultats sont donnés dans le tableau I ci-après.

**TABLEAU I**

| Epaississant | Teneur en NaCl | Point de trouble °C |
|---|---|---|
| WITCAMIDE CPA | 7 % | - 4 |
| | 8 % | - 4 |
| WITCAMIDE PPA | 7 % | - 2 |
| | 8 % | - 2 |
| Agent épaississant | 7 % | - 7 |
| selon l'exemple 1 | 8 % | - 7 |

Le point de trouble est donc nettement plus faible avec l'épaississant de l'exemple 1 qu'avec les "WITCAMIDE PPA et CPA". De plus, on a observé dans les compositions contenant les "WITCAMIDE PPA et CPA", la formation de cristaux au vieillissement ; avec l'agent épaississant, selon l'invention, on n'a pas observé ce phénomène.

### Exemple 3 - Préparation d'un liquide de lavage pour vaisselle

On a préparé un liquide détergent pour vaisselle à 10 % de matières actives ayant la formulation suivante (en % en poids) :
- Tensioactif à base d'alkylbenzène sulfonate de sodium linéaire, vendu sous la dénomination commerciale "SULFRAMINE 1230" par la société "WITCO" 23,8
- Tensioactif à base d'alkyléthersulfate de sodium vendu sous la dénomination commerciale "NEOPON LOS/NF" par la société "WITCO" 10,2
- Agent épaississant 0 ; 0,5 et 1
- Chlorure de sodium 0,5 à 3
- Eau de ville qsp 100

L'agent épaississant utilisé est, soit celui obtenu à l'exemple 1, soit, à titre comparatif, le diéthanolamide de coprah, et le monoisopropanolamide de coprah vendu sous la marque "WITCAMIDE CPA" par la société "WITCO".

On a mesuré en mPa.s la viscosité à 20° C ± 1°C à l'aide du viscosimètre BROOKFIELD RVT.

On a mesuré en ml de mousse le pouvoir moussant immédiat et après 5 mn d'une solution à 0,1 % de matières sèches des formulations dans de l'eau de ville.

Le point de trouble en °C a été déterminé et l'aspect du détergent obtenu à 20° C a été observé.

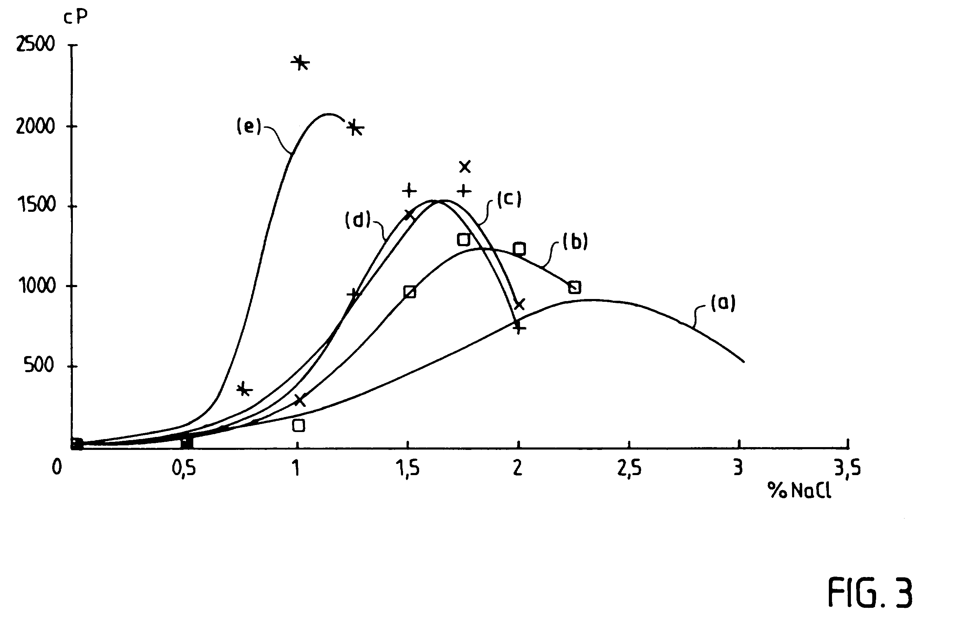

Les résultats sont donnés dans le tableau II ci-après et sur la figure 3. Sur la figure 3, la courbe (a) correspond aux viscosités obtenues avec des quantités croissantes de NaCl sans agents épaississants, la courbe (b) avec 0,5 % de diethanolamide de coprah, la courbe (d) avec 0,5 % de l'agent épaississant de l'exemple 1, la courbe (c) avec 1% de diéthanolamide de coprah et la courbe (e) avec 1 % de l'agent épaississant de l'exemple 1. Sur ces courbes la teneur en NaCl est donnée en abscisse et la viscosité en mPa.s en ordonnée.

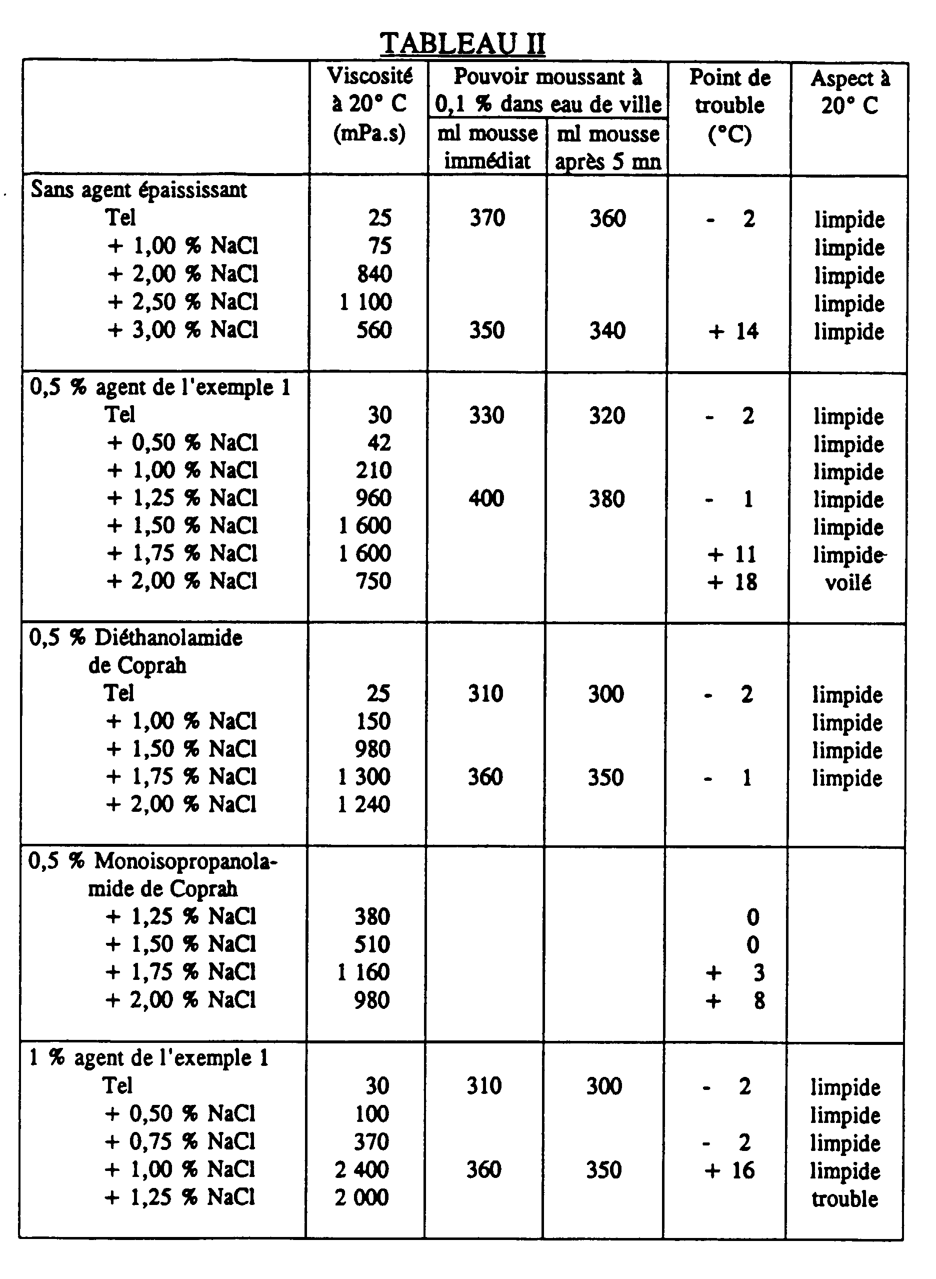

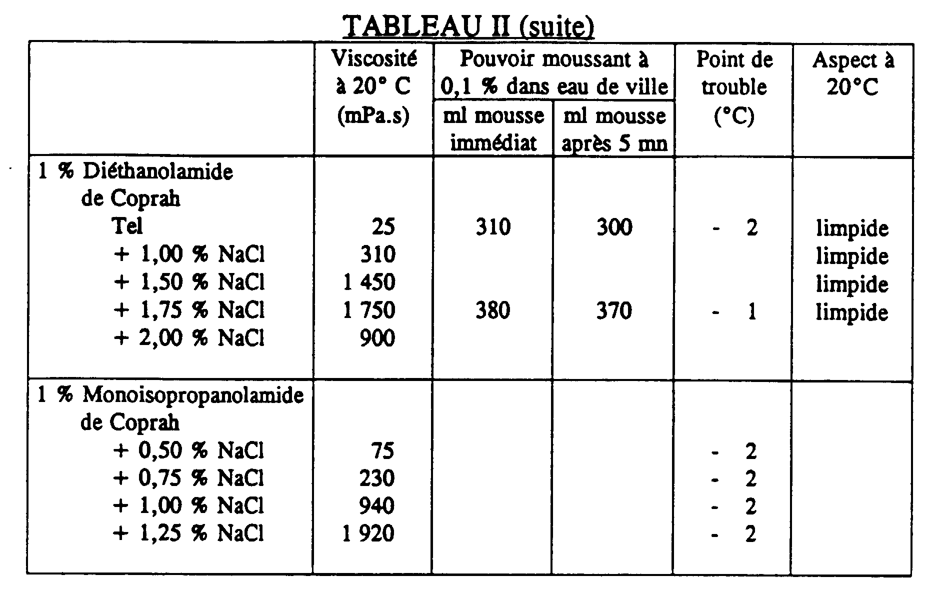

Ce tableau II et la figure 3 montrent qu'avec l'agent épaississant de l'exemple 1, on obtient une viscosité maximale plus élevée pour des teneurs en NaCl plus faibles qu'avec le diéthanolamide de coprah et le monoisopropanolamide de coprah.

## Revendications

1. Utilisation comme agent épaississant, dans une composition contenant au moins un agent tensioactif, d'une quantité efficace de l'amide de formule : 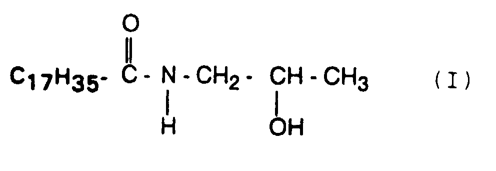 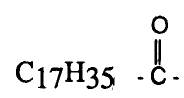 étant un radical isostéaroyle.

2. Utilisation selon la revendication 1, caractérisée par le fait que l'agent épaississant contient au moins 90 % en poids de l'amide de formule (I).

3. Utilisation selon l'une des revendications 1 ou 2, caractérisée par le fait que l'agent épaississant répond aux spécifications suivantes :
- Aspect à 25° C liquide limpide à légèrement trouble
- Densité à 40° C 0,900 - 0,908
- Viscosité à 40° C 310 à 330 mPa.s
- Couleur à 40°C (mesurée en unités Gardner) ≤ 5
- Indice d'acide (en mg KOH/g) ≤ 5
- Teneur en amine libre (en % en poids) ≤ 1
- Teneur en estéramide (en % en poids) ≤ 5
- pH mesuré en solution à 1 % en poids dans un mélange eau/isopropanol (50/50 en volume) 8 à 9
- Teneur en eau ≤ 0,5

4. Composition liquide ou pâteuse contenant au moins un agent tensioactif et au moins une quantité efficace d'un amide d'acide gras comme agent épaississant, caractérisée par le fait que l'agent épaississant est un agent épaississant selon les revendications 1 à 3.

5. Composition selon la revendication 4, caractérisée par le fait qu'elle contient un sel minéral.

6. Composition selon la revendication 5, caractérisée par le fait que la quantité de sel minéral est choisie pour optimiser la viscosité selon la quantité d'amide de formule (I) présente dans la composition.

7. Composition selon l'une des revendications 5 ou 6, caractérisée par le fait que le sel minéral est un chlorure ou un sulfate alcalin ou alcalino-terreux.

8. Composition selon la revendication 7, caractérisée par le fait qu'elle contient de 0,25 à 10 % en poids de chlorure de sodium.

9. Composition selon l'une des revendications 4 à 8, caractérisée par le fait que l'(les) agent(s) tensioactif(s) est (sont) choisi(s) dans le groupe formé par :
- les alkylsulfates alcalins, d'ammonium, de magnésium, ou d'alcanolamines ;
- les alkyléthersulfates alcalins, d'ammonium, de magnésium ;
- les alkylbenzène sulfonates alcalins ou d'alcanolamines ;
- les alphaoléfine sulfonates alcalins ;
- les alkyl amidosulfosuccinates alcalins ;
- les alkyl sulfosuccinates de sodium ;
- les savons de sodium, potassium ou triéthanolamine ;
- les bétaïnes et sulfobétaïnes ;
- les tensioactifs amphotérés dérivés d'imidazoline ;
- les alkylpolyglycoéthers ;
- les alkylarylpolyglycoéthers ;
- les esters gras de polyols.

10. Composition selon l'une des revendications 4 à 9, caractérisée par le fait qu'elle contient de 0,1 à 2,5 % en poids d'agent épaississant selon l'une des revendications 1 à 3.

11. Composition selon les revendications 8 et 10 prises simultanément, caractérisée par le fait qu'elle contient de 0,5 à 8 % en poids de chlorure de sodium pour une quantité d'amide de formule (I) comprise entre 0,1 et 2,5 % en poids.

## Claims

1. Use as a thickening agent, in a composition comprising at least one surface-active agent, of an effective amount of the amide of formula: 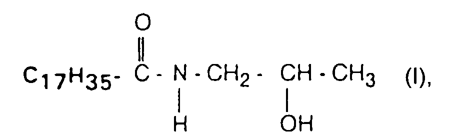 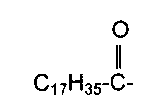 being an isostearoyl radical.

2. Use according to claim 1, characterised in that the thickening agent contains at least 90 % by weight of the amide of formula (I).

3. Use according to either claim 1 or claim 2, characterised in that the thickening agent meets the following specifications:
- Appearance at 25°C clear to slightly turbid liquid
- Density at 40°C 0.900 - 0.908
- Viscosity at 40°C 310 to 330 mPa.s
- Colour at 40°C (measured in Gardner units) ≤ 5
- Acid value (in mg KOH/g) ≤ 5
- Free amine content (in % by weight) ≤ 1
- Amide ester content (in % by weight) ≤ 5
- pH measured in a 1% by weight solution in a water/isopropanol mixture (50/50 by volume) 8 to 9
- Water content ≤ 0.5

4. Liquid or pasty composition comprising at least one surface-active agent and at least an effective amount of a fatty acid amide as thickening agent, characterised in that the thickening agent is a thickening agent according to claims 1 to 3.

5. Composition according to claim 4, characterised in that it comprises a mineral salt.

6. Composition according to claim 5, characterised in that the amount of mineral salt is so selected as to optimise the viscosity according to the amount of amide of formula (I) present in the composition.

7. Composition according to either claim 5 or claim 6, characterised in that the mineral salt is an alkali metal or alkaline-earth metal chloride or sulphate.

8. Composition according to claim 7, characterised in that it contains from 0.25 to 10 % by weight of sodium chloride.

9. Composition according to any one of claims 4 to 8, characterised in that the surface-active agent(s) is (are) selected from the group formed by:
- alkali metal, ammonium, magnesium or alkanolamine alkyl sulphates;
- alkali metal, ammonium or magnesium alkyl ether sulphates;
- alkali metal or alkanolamine alkyl benzenesulphonates;
- alkali metal alpha-olefin sulphonates;
- alkali metal alkyl amidosulphosuccinates;
- sodium alkyl sulphosuccinates;
- soaps of sodium, potassium or triethanolamine;
- betaines and sulphobetaines;
- amphoteric surface-active agents derived from imidazoline;
- alkyl polyglycol ethers;
- alkylaryl polyglycol ethers; and
- fatty esters of polyols.

10. Composition according to any one of claims 4 to 9, characterised in that it contains from 0.1 to 2.5 % by weight of thickening agent according to any one of claims 1 to 3.

11. Composition according to claims 8 and 10 taken simultaneously, characterised in that it contains from 0.5 to 8 % by weight of sodium chloride for an amount of amide of formula (I) from 0.1 to 2.5 % by weight.

## Patentansprüche

1. Verwendung einer wirksamen Menge eines Amids der Formel: 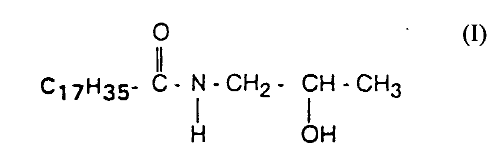 in der
C₁₇H₃₅-CO- ein Isostearylrest ist,
als Verdickungsmittel in einer mindestens ein oberflächenaktives Mittel enthaltenden Zubereitung.

2. Verwendung gemäß Anspruch 1, dadurch gekennzeichnet, daß das Verdickungsmittel mindestens 90 Gew.-% des Amids der Formel (I) enthält.

3. Verwendung gemäß einem der Ansprüche 1 oder 2, dadurch gekennzeichnet, daß das Verdickungsmittel den folgenden Anforderungen entspricht:
- Aussehen bei 25 °C: klare Flüssigkeit mit leichter Trübung
- Dichte bei 40 °C: 0,900 - 0,908
- Viskosität bei 40 °C: 310 bis 330 mPa · s
- Farbe bei 40 °C (gemessen in Gardnereinheiten): ≤ 5
- Säureindex (in mg KOH/g): ≤ 5
- Gehalt an freiem Amin (in Gew.-%): ≤ 1
- Gehalt an Esteramid (in Gew.-%): ≤ 5
- pH, gemessen mit einer 1 gew.-%igen Lösung in einer Wasser/Isopropanol-Mischung (50/50 Volumenanteile): 8 bis 9
- Wassergehalt: ≤ 0,5

4. Flüssige oder pastöse Zubereitung, enthaltend mindestens ein oberflächenaktives Mittel und mindestens eine wirksame Menge eines Fettsäureamids als Verdickungsmittel, dadurch gekennzeichnet, daß es sich bei dem Verdickungsmittel um ein Verdickungsmittel gemäß einem der Ansprüche 1 bis 3 handelt.

5. Zubereitung gemäß Anspruch 4, dadurch gekennzeichnet, daß sie ein Mineralsalz enthält.

6. Zubereitung gemäß Anspruch 5, dadurch gekennzeichnet, daß die Menge an Mineralsalz gewählt ist, um die Viskosität für die Menge an Amid der Formel (I), das in der Zubereitung vorhanden ist, zu optimieren.

7. Zubereitung gemäß einem der Ansprüche 5 oder 6, dadurch gekennzeichnet, daß das Mineralsalz ein Alkali- oder Erdalkalichlorid oder -sulfat ist.

8. Zubereitung gemäß Anspruch 7, dadurch gekennzeichnet, daß sie 0,25 bis 10 Gew.-% an Natriumchlorid enthält.

9. Zubereitung gemäß einem der Ansprüche 4 bis 8, dadurch gekennzeichnet, daß ein oder ggf. mehrere oberflächenaktive(s) Mittel aus der Gruppe ausgewählt ist bzw. sind, die besteht aus:
- Alkali-, Ammonium-, Magnesium- oder Alkanolaminalkylsulfaten,
- Alkali-, Ammonium- oder Magnesiumalkylethersulfaten,
- Alkali- oder Alkanolaminalkylbenzolsulfonaten,
- Alkali-α-olefinsulfonaten,
- Alkalialkylamidosulfosuccinaten,
- Natriumalkylsulfosuccinaten,
- Natrium-, Kalium- oder Triethanolaminseifen,
- Betainen oder Sulfobetainen,
- von Imidazolin abgeleiteten amphoteren Tensiden,
- Alkylpolyglycoethern,
- Alkylarylpolyglycoethern und
- Fettestern von Polyolen.

10. Zubereitung gemäß einem der Ansprüche 4 bis 9, dadurch gekennzeichnet, daß sie 0,1 bis 2,5 Gew.-% an Verdickungsmittel gemäß einem der Ansprüche 1 bis 3 enthält.

11. Zubereitung gemäß den Ansprüchen 8 und 10 zusammengenommen, dadurch gekennzeichnet, daß sie 0,5 bis 8 Gew.-% an Natriumchlorid auf eine zwischen 0,1 und 2,5 Gew.-% liegende Menge an Amid der Formel (I) enthält.
